# EUROPEAN PATENT SPECIFICATION

(11) **EP 0 633 760 B1**
(45) Date of publication and mention of the grant of the patent: **23.09.1998**
(21) Application number: 93907595.8
(22) Date of filing: 19.03.1993
(51) Int. Cl.: A61F 13/56

(54) **ADHESIVE/MECHANICAL FASTENER SYSTEMS FOR DISPOSABLE ABSORBENT ARTICLES**
ADHESIVES/MECHANISCHES BEFESTIGUNGSSYSTEM FÜR ABSORBIERENDE EINWEGARTIKEL
SYSTEMES D'ATTACHE ADHESIFS/MECANIQUES POUR ARTICLES ABSORBANTS JETABLES

(30) Priority: 02.04.1992 EP 92200944
(43) Date of publication of application: 18.01.1995
(73) Proprietor: THE PROCTER & GAMBLE COMPANY, Cincinnati, Ohio 45202 (US)
(72) Inventor: BUELLESFELD, Dieter, D-5350 Euskirchen-Flamersheim (DE); LANDVOGT, Brigitte, D-5350 Euskirchen-Kirchheim (DE)
(74) Representative: Bottema, Johan Jan
(86) International application number: US9302529
(87) International publication number: WO9319712

(56) References cited:
- EP-A- 0 080 647
- EP-A- 0 321 232
- EP-A- 0 324 578
- US-A- 4 869 724
- US-A- 5 108 384

## Description

### Field of the invention

The present invention relates to tape tab fastener systems for disposable absorbent articles, more particularly to tape tab fastener systems combining mechanical and adhesive tape tab fastener means in a design with improved handling characteristics, improved construction stability and which is compatible with compression packaging.

### Background of the invention

The use of tape tab fastener systems for securing the corners of disposable absorbent articles such as diapers or incontinents briefs is well known in the art. Examples of adhesive tape tab fastener systems are disclosed in US Patent RE 26,151, US Patent 3,848,594 and many other publications. While adhesive tape tab fastener systems did provide secure means for fixing a disposable absorbent article around a user's waist during use they were not totally satisfactory.

Adhesive tape tab fastener systems were often only usable upon initial fixation of the disposable absorbent article while refastening was not possible. They also displayed unsatisfactory resistance against contamination.

Hence the prior art discloses many alternative fastener systems trying to solve these problems. For example mechanical fastener systems of the hook/loop type (random entanglement) have been contemplated and disclosed in many publications. In general mechanical fastener systems have the advantage of reduced or no sensitivity versus contamination for example by oils or powders. Mechanical hook/loop fastener systems also have the advantage of being reclosable after opening for example to check the soiling of absorbent articles or to correct the fitting of absorbent articles on the wearer. However, mechanical fasteners require a two part fastening system e.g. tape tabs with hooks and a landing surface with loops.

Mechanical tape tab fasteners of the hook/loop type have been disclosed for use in diapers for example in US Patent 3,110,312 and for disposable diapers in US Patent 4,259,957 or European Patent Application EP-A-235014.

After establishing the principal advantages of mechanical fasteners over adhesive tape tab fasteners, it was found that mechanical fasteners lack the ease of disposability which the adhesive tape tab fasteners provided for disposable absorbent articles. Before disposing a used disposable absorbent article the article is often rolled up upon itself. This pack is then closed by applying the adhesive tape tab onto the outside surface of the rolled up disposable absorbent article.

The advantage of disposability is supplied by adhesive tape tabs which can be attached anywhere to the disposable absorbent article. Mechanical hook/loop fasteners by design require their respective counter surface to achieve an attachment. However, when rolling a disposable diaper onto itself one of the two mechanical fastening members, usually the landing member, is inside the roll and hence unaccessable.

Attempts to resolve this disposability problem have resulted in tape tab fastener systems combining mechanical and adhesive parts. Disclosure of such adhesive-mechanical tape tab fastener systems can be found for example in European Patent Applications EP-A-321232 or EP-A-324578. Adhesive-mechanical fastener systems have the combined advantages of low sensitivity versus contamination and exceptionally good reclosure performance as well as easy disposal of the absorbent article after use. However they still exhibit certain problems which only now have been defined and which are solved by the present invention.

Most adhesive-mechanical tape tab fasteners are in principle made like regular adhesive tape tab fasteners but include a mechanical fastener member on the adhesive tape surface. The adhesive of the tape is used to join the mechanical fastener member to the adhesive-mechanical tape tab fastener. In the process of making such fasteners, the mechanical fastening member and the adhesive tape must be pressed together. The compression necessary to securely join the mechanical fastening member to the adhesive tape often exceeds the stability of the mechanical fastening member especially if it comprises protruding elements e.g. hooks. It therefore is advantageous if attaching the mechanical fastener member to an adhesive tape would allow to provide the necessary pressure upon joining them while not affecting the mechanical fastening member. This problem of providing intact mechanical fastening members while ensuring secure attachment of the mechanical fastening member to a tape tab is one of the problems addressed by the present invention.

In European Patent Application EP 349 050 and EP 425 008 packages containing flexible articles such as disposable diapers have been disclosed in which the diapers are under up to 60 % compression. These compression packages have since then become widely used, particularly since they reduce packaged volume and packaging material consumption while containing the same number of diapers as the historically used uncompressed packages. During packing very high pressures have to be exerted on the disposable absorbent articles to achieve the volume reduction necessary for making compression packages.

When having been packed into compression packages disposable absorbent articles with adhesive-mechanical tape tab fastener systems of the designs disclosed in the prior art often exhibit the problem of reduced mechanical fastening performance. It is believed that for diapers with mechanical fastening members comprising protruding elements like hooks or loops, this problem is due to the bending, weakening or destruction of at least some of the hooks or loops during compression.

Another problem of adhesive-mechanical tape tab fastener systems for disposable absorbent articles was the length of the grip-tab at the free end of the tape tab allowing to manipulate the tape tab fastener. In the case of diapers the grip-tab was either too short for the mother applying a diaper to easily grab the tape tab or, if it was long enough for the mother, the babies were also able to open the diaper.

A third problem associated with adhesive-mechanical fastener systems was the extreme accuracy required by the manufacturing equipment to ensure that the adhesive part of the adhesive-mechanical fastener system was placed such as to hold it in its folded position until the disposable absorbent article was packed.

A first objective of the present invention is to provide adhesive-mechanical tape tab fasteners for disposable absorbent articles which maintain the mechanical fastener member's integrity during the manufacturing process by preventing the required application pressure, when placing the mechanical fastening member on the adhesive tape tab, from damaging its protruding elements.

A further objective of the present invention is to provide adhesive-mechanical tape tab fastener systems for disposable absorbent articles which are more compatible with compression packaging.

It is yet another objective of the present invention to provide disposable absorbent articles having adhesive-mechanical fastener systems with reduced accuracy requirements towards the production process regarding the placement of the mechanical fastening member on the adhesive-mechanical tape tab.

And another objective of the present invention is to provide an adhesive-mechanical tape tab fastener system having the afore-mentioned advantages at no increase or even at reduced material consumption to satisfy regulatory and user demands for ecological and economical fastening systems for disposable absorbent articles.

These and other objectives of the present invention will be more readily apparent from the following description and the accompanying drawings.

### Summary of the invention

According to the present invention, the disposable absorbent article such as a baby diaper or an adult incontinent brief is provided with a mechanical-adhesive tape tab fastener system. The absorbent article has an outside surface and an inside surface. The fastener system comprises a tape tab which is joined to the disposable absorbent article in a first end region with the fastener's manufacturing region, preferably by an adhesive. The free or user's end of the tape tab comprises two fastening members : a mechanical fastening member and an adhesive fastening member.

The mechanical fastening member represents the primary closure system of the absorbent article during wearing. When the absorbent article is worn, the mechanical part of the fastening system operates by overlapping the mechanical fastening member on the tape tab onto a landing member which is designed to mechanically entangle with the mechanical fastening member of the tape tab. Particular preferred are hook type mechanical fastening members on the tape tab to be randomly entangled with loop type landing members. The landing member is disposed on the outside surface in the second end region of the absorbent article such that the disposable absorbent article can be wrapped around the wearer's hips and closed around the side of the wearer by use of the mechanical fastening member of the tape tab fastener and the landing member.

The adhesive fastening member provides the desired disposability function when the disposable absorbent article is rolled up into a neat package after use which is closed by placing the adhesive member anywhere on the outside surface of the absorbent article.

The invention relates to the improved quality of the attachment of the mechanical fastening member to the tape tab fastener. In order to use a tape tab fastener, it is usual to include a grip-tap at the outward end of the tape tab. This grip-tab provides the means to manipulate the tape tab into the appropriate position for closing the fastener system. According to the invention, the grip-tab extends over part of the mechanical fastening member without interfering with the mechanical fastening member function. The grip-tab also extends into the outward end of the tape tab thus providing an additional attachment for the mechanical member to the tape tab fastener. In this design the adhesive fastening member is disposed on the tape tab fastener between the manufacturer's region and the latterally outward end of the tape tab fastener. It is particularly beneficial and preferred that the mechanical member is attached to an adhesive tape tab fastener and that the grip-tap is provided by folding the outward end of the tape tab fastener over onto itself and onto at least part of the mechanical fastening member.

In a preferred embodiment of the present invention, in addition to the improved strength of the attachment of the mechanical fastening member to the tape tab fastener, the mechanical elements of the mechanical fastening member are protected during high compression packaging by providing them with a soft and compressable countersurface.

During transport of the disposable absorbent article the mechanical-adhesive tape tab fastener are fully folded onto the inside surfaces of the disposable absorbent article so as to not extend outside the body portion of the absorbent article. In this configuration the adhesive and mechanical fastening members rest with their active sides well protected on the body size surface of the absorbent article. In order to protect the adhesive fastening member a release tape is disposed on the inside surfaces of the absorbent article. This release tape may also provide a soft and compressible countersurface for the mechanical fastening member. However, according to the invention, the release tape only extends to an area corresponding to the adhesive fastening member. The mechanical fastening member in this preferred embodiment of the invention is provided with a soft and compressable countersurface by the inside surfaces of the absorbent article which by its design provides a soft and compliant surface.

### Brief description of the drawings

Figure 1 is a partially cut-away perspective view of a diaper embodiment incorporating the present invention.

Figure 2 is a cross-sectional view of the corner of the diaper taken along line 2-2 of Figure 1.

### Detailed description of the invention

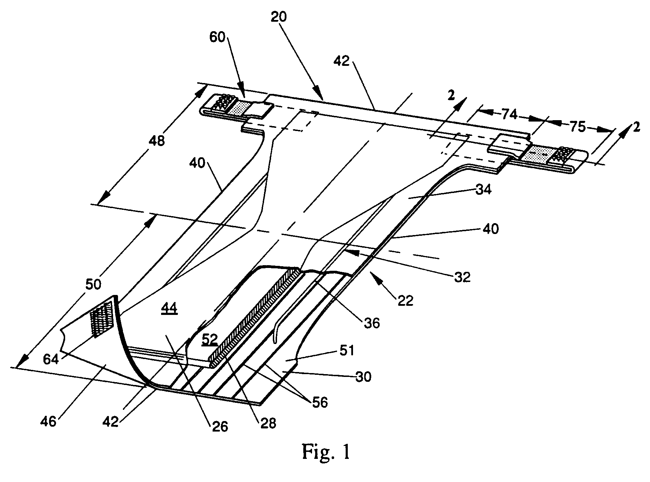

Adhesive mechanical fastener systems of the present invention are useful and beneficial when applied to disposable absorbent articles. As used herein the term "disposable absorbent articles" refers to articles which absorb and contain body exudates and more specifically refers to articles which are placed against or in the proximity of the wearer's body to absorb and contain the various exudates discharged from the body of the wearer and which are not intended to be laundered or otherwise restored or reused as an absorbent article (i.e. they are intended to be discarded after a single use and, preferably to be recycled, composted or otherwise disposed of in an environmentally compatible manner). A preferred embodiment of the disposable absorbent article of the present invention is an adult incontinence brief or a baby diaper, as shown in Figure 1 as diaper (20). As used hereinafter the term "diaper" refers to an absorbent article generally worn by infants or incontinent persons that is drawn up between the legs and fastened about the waist of the wearer. Examples of the kind of diapers to which the present invention is readily adapted are shown in US patent RE 26,151; US patent 3,860,003; 4,253,461; and 4,704,115. It will be apparent from the following description that the adhesive-mechanical fastener system illustrated and described herein, could be applied to the body portion on such diapers. On the other hand, it will be understood that the invention is not limited to any specific diaper structure or configuration, provided it has a fastening system and is compatible with the requirements of the disposable absorbent articles disclosed herein.

Referring to the drawings, it will be noted that Figure 1 is a partially cut-away perspective view of the diaper (20) according to the present invention prior to its being placed on a wearer. As can be seen in Figure 1, a preferred diaper (20) comprises an adhesive-mechanical tape tab fastener, designated generally as (60). A preferred body portion (22) comprises a liquid pervious topsheet (26), an absorbent core (28), a liquid impervious backsheet (30), and elastically contractible leg cuffs (32) comprising a side flap (34) and one or more elastic members (36). For simplicity purposes, only 1 elastic member is shown in the side flap (34). While the topsheet (26), the absorbent core (28), the backsheet (30), the side flaps (34), and the elastic members (36) may be assembled in a variety of well-known configurations. A preferred diaper configuration is shown and described generally in the above referenced US patent 3,860,003 or 4,253,461.

Figure 1 shows a preferred embodiment of the body portion (22) in which the topsheet (26), the backsheet (30) are coextensive and have length and widths dimensions generally larger than those of the absorbent core (28). The topsheet (26) is superposed to the backsheet (30) thereby forming the periphery of the body portion (22). The periphery defines the outer primeter or in other words the outer extent of the body portion (22). The periphery comprises the longitudinal side edges (40) and the first and second lateral end edges (42). In longitudinal direction the diaper (20) has a first end region (48) and a second end region (50).

The body portion (22) has an inside surface (44) and an ouside surface (46). In general, the ouside surface (46) of the diaper (20) extends from a first lateral end edge (42) to a second lateral end edge (42) of the diaper (20) and from one longitudinal side edge (40) to the other longitudinal side edge (40) of the diaper.The ouside surface (46) usually is the surface farthest from the wearer during use of the diaper (20). The backsheet (30) preferably forms most of the ouside surface (46) of the body portion (22). The inside surface (44) is that surface of the diaper (20) opposite the ouside surface (46) and in the embodiment shown in Figure 1 is preferably formed by the topsheet (26). Preferably, the inside surface (44) of the diaper (20) is coextensive with the ouside surface (46) and in general the inside surface (44) is for the greater part in contact with the wearer when the diaper (20) is used.

The diaper (20) has first and second end regions (48 and 50, respectively), extending from the first and second end edges (42) of the diaper periphery towards the lateral center line of the diaper (20). Both the first end region (48) and the second end region (50) extend a distance of about 1_{/2} of the length of the diaper (20) such that the end regions each comprise half of the diaper (20).

The absorbent core (28) of the body portion 22 may be any absorbent means which is generally compressible, conformable, non-irritating to the wearer's skin, and capable of absorbing and retaining liquids such as urine and other certain body exudates. The absorbent core (28) may be manufactured in a wide variety of sizes and shapes (e.g., rectangular, hourglass, asymmetric, T-shaped, etc.) and from a wide variety of liquid absorbent materials commonly used in diapers and other disposable absorbent articles such as comminuted wood pulp which is generally referred to as airfelt. Examples of other suitable absorbent materials include creped cellulose wadding, meltblown polymers including coform, cross-linked cellulosic fibers, tissue including tissue wraps and tissue laminates, absorbent foams, absorbent sponges, superabsorbent polymers, absorbent gelling materials or any equivalent materials or combination of materials. The configuration and construction of the absorbent core may also be varied e.g., the absorbent core may have varying caliper zones, a hydrophillic gradient, a superabsorbent gradient (as in concentration or particle size for granular superabsorbents), or lower average density and lower average basis weight acquisition zones; or may comprise one or more layers or structures. The total absorbent capacity of the absorbent core (28) should, however, be compatible with the design exudate loading and the intended use of the diaper (20). Further, the size and absorbent capacity of the absorbent core (28) may be varied to accomodate wearers ranging from infants to adults.

A preferred embodiment of the diaper (20) has an hourglass-shaped absorbent core (28). An exemplary absorbent structure for use as the absorbent core is described in US Patent 4,610,678, 4,673,402, 4,888,231. The absorbent core (28) is preferably the commercially successful absorbent member described in US Patent 4,834,735, entitled "High Density Absorbent Members Having Lower Density and Lower Basis Weight Acquisition zones". Other preferred absorbent cores are described in US Patents 4,685,915 and 4,781,710 as comprising fibrous structures having areas of different absorbent capacity, density, or liquid acquisition speed. An alternative thin absorbent core useful in the present invention may be found in US Patent 4,600,458. Another preferred absorbent core design provides for a crosslinked cellulose patch essentially without superabsorbent on top of an airfelt with superabsorbent mixture comprising more than 30% superabsorbent.

The absorbent core (28) is superposed on the backsheet (30) and is preferably joined thereto by a core attachment means (not shown) such as those well known in the art, for example, pressure-sensitive adhesives, hot melt adhesives or other adhesives; ultrasonic bonds; heat/pressure bonds, dynamic mechanical bonds; or any other suitable attachment means or combinations of these attachment means as are known in the art. For example, the absorbent core (28) may be secured to the backsheet (30) by a uniform continuous layer of adhesive, a patterned layer of adhesive, or a network of adhesive filaments such as any array of random lines, separate lines, spirals, or spots of adhesive. Adhesives which have been found to be satisfactory are preferably hot melt adhesives such as manufactured by Century Adhesives, Inc., of Columbus, Ohio and marketed under the tradename Century 5227; and by H. B. Fuller Company of St. Paul, Minnesota and marketed as HL-1258. The core attachment means preferably comprise an open pattern network of filaments of adhesive as is shown in US Patent 4,573,986 entitled "Disposable Waste-Containment Garment". An exemplary attachment means of an open pattern network of filaments comprises several lines of adhesive filaments swirled into a spiral pattern such as is illustrated by the apparatus and methods shown in US Patent 3,911,173, 4,785,996 or 4,842,666.

The backsheet (30) is impervious to liquids (e.g. urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (30) prevents the exudates absorbed and contained in the absorbent core (28) from soiling articles which contact the diaper (20) such as bedsheets and undergarments. The backsheet may thus comprise polymeric films such as thermoplastic films of polyethylene or polypropylene, or composite materials such as a film-coated nonwoven material. For economic, aesthetic, and ecological reasons, the backsheet (30) preferably has an average nominal caliper, i.e. calculated caliper, of less than about 0.051 mm, more preferably a calculated caliper of from 0.020 mm to 0.036 mm.
Preferably, the backsheet (30) is a flexible polyethylene film. As used herein the term "polyethylene" film refers to films which are essentially made of polyethylene, however, it is understood that polyethylene film will contain a variety of additives to provide characteristics like opacity, strength requirements, color, or any other desired characteristic that can be achieved through adding minor amounts of other substances than polyethylene into the films. The total amount of additives should be less than 45%, preferably less than 15%, by weight of film materials. Particularly for opacity of the film, titanium dioxide is commonly used in a range of 2-6%, preferably 3.5-4.8%, by weight of the film. Exemplary films for use as the backsheet of the present invention are manufactured by Tredegar industries, Inc. of Terre Haute, Indiana, USA or BP-Chemical PlasTec Rotbuchenstrasse 1 D- 8000 MÜNCHEN, Germany.

The backsheet (30) is preferably textured to provide a more clothlike appearance. Further, the backsheet (30) may also permit vapors to excape from the the absorbent core (28) while still preventing exudates from passing through the backsheet (30) by ,for example, being supplied with microapertures as described, for example, in US Patent 4,681,793. The backsheet may also be biodegradable such as the film disclosed in Patent Specification WO 93/00116 entitled "Disposable Absorbent Articles with Biodegradable Backsheets".

The size of the backsheet (30) is dictated by the size of the absorbent core (28) and the exact diaper design selected. In a preferred embodiment, the backsheet (30) has a modified hourglass shape extending beyond the absorbent core a minimum distance of at least 1.3 cm to 2.5 cm for baby diapers and 1.3 cm to 6 cm for adult incontinent briefs around the entire diaper periphery.

The topsheet (26) of the body portion (22) of the present invention is compliant, soft feeling, and non-irritating to the wearer's skin. Further, the topsheet (26) is liquid pervious permitting liquids (e.g. urine) to readily penetrate through its thickness. A suitable topsheet (26) may be manufactured from a wide range of materials, such as porous foams, reticulated foams, apertured films; or woven or nonwoven webs of natural fibers (e.g. wood or cotton fibers), synthetic fibers (e.g. polyester or polypropylene fibers) or from a combination of natural and synthetic fibers. Preferably, it is made of a material that isolates the wearer's skin from liquids retained in the absorbent core (28).

There are a number of manufacturing techniques which may be used to manufacture the topsheet (26). For example, the topsheet (26) may be a non-woven web of fibers. When the topsheet comprises a nonwoven web, the web may be spunbonded, carded, wet-laid, meltblown, hydroentangled, hydroformed, combinations of the above, or the like. An exemplary topsheet (26) is carded and thermally bonded by means well known to those skilled in the fabric art and comprises staple length polypropylene fibers having a denier of about 2.2 and has a basis weight from about 15 to about 30 grams per square meter. As used herein, the term "staple length fibers" refer to those fibers having a length of at least about 16 mm. A suitable topsheet is manufactured for example by Veratec, Inc., a Division of International paper Company, of Walpole, Massachussets under the designation P-8. A topsheet particularly preferred for incontinent briefs of the present invention, comprises a formed thermoplastic film such as that described in US Patent 3,929,135 entitled "Absorptive Structure Having tapered Capillaries".

The topsheet (26) and the backsheet (30) are joined together in any suitable manner as is well known in the diaper manufacturing art. As used herein, the term "joined" encompasses configurations whereby the topsheet (26) is directly joined to the backsheet (30) by affixing the topsheet (26) directly to the backsheet (30), and configurations whereby the topsheet (26) is indirectly joined to the backsheet (30) by affixing the topsheet (26) to intermediate members (e.g. absorbent core (28)) which in turn are affixed to the backsheet (30). In a preferred embodiment, the topsheet (26) and the backsheet (30) are joined directly to each other in the diaper periphery by a flap attachment means such as an adhesive or any other attachment means as is known in the art. In general, the core attachment means that affixes the absorbent core (28) to the backsheet (30) is the same means as the flap attachment means (56) that affixes the topsheet (26) to the backsheet. Thus, for example, a uniform continuous layer of adhesive, a patterned layer of adhesive, an array of separate lines, spirals, or spots of adhesive such as a network of adhesive filaments such as shown in US Patent 4,573,986, may be used.

The diaper (20) preferably further comprises elasticized leg cuffs (32) for providing improved containment of liquids and other body exudates. Each elasticized leg cuff (32) may comprise several different embodiments for reducing the leakage of body exudates in the leg regions. (The leg cuff can be and is sometimes also referred to as leg bands, side flaps, barrier cuffs, or elastic cuffs.) US Patent 3,860,003 entitled "Contractable Side Portions for a disposable diaper" describes a disposable which provides a contractible leg opening having a side flap and elastic members to provide an elasticized leg cuff (gasketing cuff). US Patent 4,909,803 entitled "Disposable absorbent article having elasticized flaps" describes a disposable diaper having "stand-up" elasticized flaps (barrier cuffs) to improve the containment of the leg regions. US Patent 4,695,278 entitled "Absorbent article having dual cuffs" describes a disposable diaper having dual cuffs including a gasketing cuff and a barrier cuff. US patent 4,704,115 entitled "Disposable waist Containment garment" discloses a disposable diaper or incontinent garment having side-edge-leakage-guard gutters configured to contain free liquids within the garment. While each elasticized leg cuff (32) may be configured so as to be similar to any of the leg bands, side flaps, barrier cuffs, or elastic cuffs described by above disclosures, it is common for the present invention that each elasticized leg cuff (32) comprises a side flap (34) and one or more elastic members (36) such as shown in Figure 1.

The diaper may also further comprise an elastic waist feature (not shown in Figure 1) that provides improved fit and containment or any other features typically provided on diapers or incontinent garments as are known in the art. An exemplary elasticized waist feature is described in US patent 4,515,595.

The diaper (20) is provided with an adhesive-mechanical fastening system for forming a side closure on each side of the diaper (20). Thus, the diaper (20) is fitted to the wearer and the first end region (48) and the second end region (50) are maintained in an overlapping configuration when the diaper (20) is worn such that lateral tensions are maintained around the circumference of the diaper to maintain the diaper on the wearer.

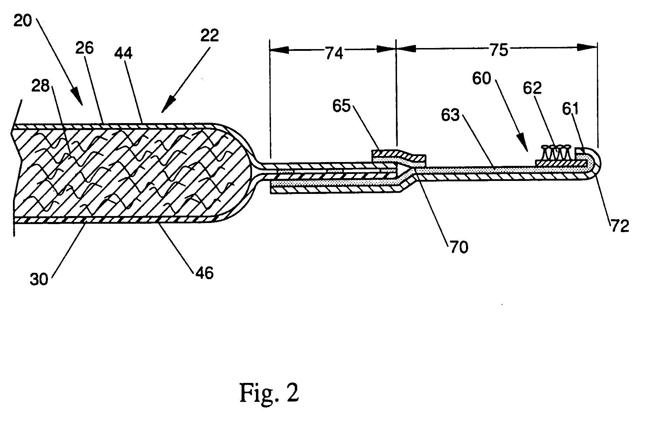

According to the present invention as shown in Figure 1, the fastening system comprises two elements, an adhesive-mechanical tape tab fastener (60) and a landing member (64) which are joined to each other during use. The tape tab has a manufacturer's region (74) and a user's region (75). The tape tab fastener (60) is disposed in the first lateral end region (48) such that the manufacturer's region (74) is joined to the absorbent article (20) and the user's region (75) extends beyond the longitudinal side edge (40) of the absorbent article (20). The user's region (75) has a proximal region which comprises an adhesive fastening member (63) and it has a distal region which comprises a mechanical fastening member (62) and a grip-tab (61). As shown in Figure 2, the manufacturer's region (74) of the tape tab fastener (60) is preferably joined to the ouside surface (46) of the absorbent article (20). However, it can also be sandwiched between the topsheet (26) and the backsheet (30) of the absorbent article (20).

As shown in Figure 2 in a particular preferred embodiment of the present invention, the tape tab fastener (60) further comprises a release tape (65). The release tape is disposed such that the tape tab fastener (60) in its package configuration, that is when the tape tab fastener (60) is folded along the separating line of the manufacturer's region (74) and the user's region (75) onto itself towards the inside surface (44) of the absorbent article (20), provides a release surface to the adhesive fastening member (63) in order to protect the adhesive fastening member (63) from contamination and in addition to maintain the folded tape tab fastener (60) in its packaged position. Preferably, the release tape (65) is disposed such that the engaging elements of the mechanical fastening member (62) are not in contact with the release surface of the release tape (65) when the tape tab fastener (60) is in its packaged configuration.

In a preferred embodiment the release tape (65) extends slightly beyond the longitudinal side edge (40) into the user's region (75) of the tape tab fastener (60) and is joined, preferably by adhesive to adhesive attachment in a Y -bond (70) to the adhesive fastening member (63) of the tape tap fastener(60).

For defect free and convenient handling upon use of an absorbent article it is further preferred that in the packaged position the adhesive contact area between the adhesive fastening member (63) and the release tape (65) does not extend all the way to the laterally inward end of the release tape (65). The preferred distance between the latterally inward ends of the adhesive contact area and the release tape is from 3 mm to 10 mm, most preferred 5 mm to 7 mm. A distance of 6 mm has been found to provide satisfactory performance.

The grip-tab (61) can be provided by an additional piece of material which is disposed from the laterally outward edge (72) of the user's region (75) and extends over at least part of the mechanical fastening member (62). Preferably the grip-tab (61) is formed by folding the laterally outward edge (72) of the user's region (75) back onto itself and over part of the mechanical fastening member (62). The overlap of the grip-tab (61) with the mechanical fastening member (62) provides the mechanical fastening member (62) with an additional attachment to the tape tab fastener (60).

Yet in another preferred embodiment as shown in Figure 2 of the tape tap fastener (60) of the present invention, the tape tab fastener has a continuous adhesive tape material extending from the manufacturer's region(74) to the user's region (75) thereby providing the adhesive fastening member (63) without additional materials and comprises a grip-tap (61) which is folded over onto itself and onto the mechanical fastening member (62). It can also comprise in addition the preferred release tape and Y-bond. Y-bond constructions are shown and explained in detail in US Patent 3,848,594 entitled "Tape fastening system for disposable diapers"

The fastening-landing member (64)for corporation with the mechanical fastening members is disposed in the second lateral end region (50) and joined to the ouside surface (46) of the diaper (20). The landing member (64) can be a unitary material to which all tape tab fasteners of the diaper (20) are attached during use. However, as indicated in Figure 1, the landing member (64) can also be provided in smaller, more economical and ecological sizes depending on the use for which the absorbent article is designed.

The tape tab fastener (60) comprises a tape backing material which can be any of the tape backing materials well known in the art. For example, polyester films, polypropylene films, paper backings, or other materials which provide the required strength to be useful as part of a tape tab fastener (60) during use of a diaper (20) are suitable for use as the tape backing material. Particularly, tape backing materials of polypropylene film having a caliper of about 0.15 mm have been found to perform satisfactorily. The same materials used for the tape tab backing material can be used for the release tape (65). In order to perform its release function, the side of the release tape that is not attached to the inside surface (44) can be coated with a release agent, preferably with a silicone release coating, which is well known in the art.

The tape tab fastener has an adhesive fastening member (63) having a layer of adhesive coated onto the tape backing material. (As used herein, the term "coated" is not to be limited to any specific technique or method for applying the adhesive onto the tape backing material). the adhesive fasting member may use any suitable adhesive that provides the desired attachment strength required for its disposability function. The composition of the adhesive for the adhesive fastening member (63), is not as important as the properties of the overall tape tab fastener (60). The adhesive can, for example, be a hot melt adhesive which is coated onto the tape backing by any of the well known hot melt coating processes (e.g. by a slot coating process). Alternatively, the adhesive can be supplied in a solvent coating process. Preferably, the adhesive is an elastomeric pressure-sensitive adhesive. It is particularly preferred that such an adhesive material comprises a tackified rubber elastomer. In accordance with the present invention, it has been found that tapes such as are manufactured by the Minnesota Mining and Manufacturing Company, St. Paul, Minnesota, under the designation XPF 14.43.0, Y-9376 or Y-9030 for the tape backing material and XPF 1.42.34 for the adhesive, have been found to provide satisfactory performance in the tape tab fasteners of the present invention.

The joining of the manufacturer's region (74) to the body portion (22) can be provided by mechanical or preferably by adhesive means. In a preferred embodiment, the tape backing of the tape tab fastener (60) is coated on its whole surface with a layer of adhesive. This adhesive, thus, not only provides the adhesive fastening member (63) but also the attachment to permanently adhere the manufacturer's region (74) to the ouside surface (46), preferably to the backsheet (30). If the adhesive further is coated also laterally outside the adhesive fastening member (63), it provides the means for joining the mechanical fastening member (62) to the tape backing and the possibility to fold over the outside edge of the tape tab fastener (60) onto itself thus forming the grip-tap (61).

Even so less preferred versus adhesive means to secure the manufacturer's region (74) to the body portion (22) mechanical means are well known in the art and can be used. For example ultrasonic bonding or welding by use of heat and/or pressure energy are useful in this context.

The mechanical fastening member (62) of the adhesive-mechanical tape tab fastener (60) preferably comprises a hook fastening material. As used herein the term "hook fastening material" is used to designate a material having engaging elements. The hook fastening material (62) may also be referred to as male fastener. It should be understood that the use of the term "hook" should be non-limiting in the sense that the engaging elements may comprise any shapes as are known in the art as long as they are adapted to engage a complementary second fastening element which is comprised in the landing member (64). The hook fastening material preferably comprises a base having a first surface and a second surface and a plurality of engaging elements extending from the first surface of the base. It is particularly preferred, as shown in Figure 2, that the mechanical fastening member (62) has the engaging elements extend only from the base where the grip-tab does not cover the mechanical fastening member (62).

The preferrred hook fastening material (62) of the present invention is intended to engage a fibrous element of a loop fastening material so as to provide a secure fastening device. Thus, hook fastening material (62) may be manufactured from a wide range of materials. Suitable materials include nylon, polyester, polypropylene or any combination of these materials. A suitable hook fastening material(62) comprises a number of shaped engaging elements projecting from an extruded film backing or a woven backing such as the commercially available material designated Scotchmate Brand (TM) number FJ 3402 available from Minnesota Mining and Manufacturing Company, St. Paul, Minnesota, USA. Alternatively the engaging elements may have any shape such as hooks, "Ts", mushrooms or any other shape as are well known in the art. A particular preferred hook fastening material (62) is described in European Application EP-A-276970.

The landing member (64) preferably comprises a fastening element which is engagable with the mechanical fastening member (62). The fastening elements of the landing member (64) may be manufactured from a wide range of materials and configurations capable of securily engaging the mechanical fastening member (62). For example it may comprise identical complementary elements with the mechanical fastening member (62) or distinct complementary elements with the mechanical fastening member (62). As used herein, the term identical complementary elements is used to define mechanical fastening systems wherein the engaging elements of the two fastening members of the mechanical fastener comprise the same configuration or structure that are interlocking. Examples of such systems are described in US Patent 4,322,875 or 4,701,179. The term "distinct complementary elements" is used herein to designate a system wherein the mechanical fastening member (62) is different from the landing member (64) but is engagable such as buttons and holes a hook fastening material and a loop fastening material or a male member and a female member. Preferably, the landing member (64) comprises a hook fastening material or a loop fastening material depending upon whether the mechanical fastening member (62) is a loop fastening material or a hook fastening material. The landing member (64) preferably comprises a plurality of fibrous elements as loop fastening material.

The loop fastening material of the landing member (64) provides a plurality fibre elements that engage the engaging elements of the hook fastening materials (62). The loop fastening material may be manufactured from a wide range of materials to provide fibre elements, preferably loops. Such suitable materials include nylon, polyester, polypropylene, or any combination of these materials. A suitable loop fastening material for a landing member (64) comprises a number of fibre loops projecting from a woven backing such as the commericially avaible material designated Scotchmate brand (TM) nylon, woven loop number SJ 3401 available from Minnesota Mining and Manufacturing Company, St. Paul, Minnesota, USA. A preferred loop fastening material comprises a tricot knit fabric having a plurality of nylon filament loops projecting from a backing of nylon such as the commercially available material designated "Guilford No 16110" (TM) available from Guilford Mills of Greenborough, North Carolina, USA. Alternatively, the loop fastening material of the landing member (64) may be a non-woven fabric or any other type of fibre material or loop material which are well known in the art. An inexpensive loop fastening material and a method of making such a loop fastening material is described in European Patent Application EP-A-289198.

The landing member (64) or as shown in Figure 1 the landing members (64) as well as the mechanical fastening member (62) can be joined in their respective places on the diaper (20) by any of the afore-mentioned mechanical or adhesive means.

In use the diaper (20) is applied to the wearer by positioning the first end region (48) under the wearer's back and drawing the remainder of the diaper (20) between the legs of the wearer so that the second end region (50) is positioned across the front of the wearer. Mechanical fastening member (62) of the fastening tape tabs (60) are then secured to the landing members (64) positioned on the outside surface (46) of the second end region (50) so that the mechanical fastening member (62) will engage the fastening elements with the fastening elements of the landing member (64) and the adhesive fastening member (63) is not exposed to contamination by lightly adhering to the outside surface (46) of the diaper (20) or to the landing member (64) whichever is disposed below it. After the diaper (20) has been soiled, the diaper (20) is removed from the wearer. The side panels (51) in the second end region (50) may then be folded in and the body portion (22) beginning with the second end region (50) folded or rolled into a structure with the adhesive-mechanical tape tab fastener (60) still extending outwardly from the rolled up diaper (20). The adhesive fastening member (63) is then secured to either the other tape tab fastener (60) or to the backsheet (30) so as to secure the diaper (20) in its rolled up configuration such that it may be easily and conveniently disposed in a waist reciptical.

## Claims

1. A disposable absorbent article having an inside surface (44) and an outside surface (46), longitudinal side edges (40) in the longitudinal direction, a first and a second lateral end edge (42) in the lateral direction, a first end region (48) and a second end region (50), said absorbent article comprising:
- a body portion (22) defined by said longitudinal side edges (40) and said first and second lateral end edges (42);
- an adhesive-mechanical fastening tape tab (60) having a manufacturer's region (74) and a user's region (75); said tape tab (60) being disposed in said lateral first end region (48) such that said manufacturer's region (74) is joined to said absorbent article (20) and said user's region (75) extends beyond said longitudinal side edge (40) of said absorbent article (20); said user's region (75) having a proximal region to said longitudinal side edge (40) and a distal region to said longitudinal side edge (40); said proximal region comprising an adhesive fastening member (63) and said distal region comprising a mechanical fastening member (62) and a grip tab (61), said adhesive fastening member (63) and said mechanical fastening member (62) each having their fastening surface oriented in the same direction as said inside surface (44); said grip tab (61) being disposed at laterally outward edge (72) of said user's region (75);
- a mechanical fastening landing member (64) for co-operation with said mechanical fastening member (62) of said adhesive-mechanical fastening tape tab (60), said landing member (64) being disposed in said lateral second end region (50) and joined to said outside surface (46) of said absorbent article (20); and
- said grip tab (61) extending over part of distal region of said user's region (75) and provided by folding said laterally outward edge (72) of user's region (75) back onto itself, characterised in that
said grip tab (61) extends over part of said mechanical fastening member (62).

2. Disposable absorbent article (20) according to claim 1 wherein said grip tab (61) is continuous with said proximal region of said user's region (75).

3. Disposable absorbent article (20) according to any of the preceding claims wherein said absorbent article (20) further comprises a release tape (65), said release tape (65) being disposed on said inside surface (44) of said absorbent article (20) to cooperate with said adhesive-mechanical fastening tape tab (60) during storage and transport of said absorbent article (20).

4. Disposable absorbent article (20) according to claim 3 wherein said release tape (65) is joined to said inside surface (44) of said absorbent article (20) by an adhesive.

5. Disposable absorbent article (20) according to any of the preceding claims wherein said proximal region and said distal region of said user's region (75) are continuous and have a continuous adhesive layer to form said adhesive fastening member (63) in said proximal region, and said mechanical fastening member (62) being joined to said adhesive-mechanical fastening tape tab (60) by said continuous adhesive layer.

6. Disposable absorbent article (20) according to claim 5 wherein said user's region (75) and said manufacturer's region (74) are continuous, said user's region (75) and said manufacturer's region (74) being continuous and having a continuous adhesive layer, said manufacturer's region (74) being secured to said outside surface (46) of said absorbent article (20) by said continuous adhesive layer.

7. Disposable absorbent article (20) according to any of the preceding claims wherein said mechanical fastening member (62) comprises hook type engaging means and said mechanical fastening landing member comprises loop type engaging means to engage with said hook type engaging means during use of said absorbent article (20).

8. Disposable absorbent article (20) according to any of the preceding claims wherein said mechanical fastening landing member (64) is joined to said outside surface (46) of said absorbent article (20) by an adhesive and said mechanical fastening landing member (64) is provided as a unitary piece.

9. Disposable absorbent article (20) according to any of the preceding claims wherein said disposable absorbent article (20) is a disposable absorbent diaper (20).

## Patentansprüche

1. Absorbierender Einwegartikel mit einer Innenfläche (44) und einer Außenfläche (46), Längsseitenkanten (40) in Längsrichtung, einer ersten und einer zweiten seitlichen Stirnkante (42) in seitlicher Richtung, einem ersten Endbereich (48) und einem zweiten Endbereich (50), wobei der absorbierende Artikel umfaßt:
- einen Körperbereich (22), der durch die Längsseitenkanten (40) und die erste und die zweite seitliche Stirnkante (42) begrenzt ist;
- ein haft-mechanischer Befestigungsbandstreifen (60) mit einem Herstellerbereich (74) und einem Nutzerbereich (75); wobei der Bandstreifen (60) in dem seitlichen ersten Endbereich (48) derart angeordnet ist, daß der Herstellerbereich (74) mit dem absorbierenden Artikel (20) verbunden ist und sich der Nutzerbereich (75) über die Längsseitenkante (40) des absorbierenden Artikels (20) hinaus erstreckt; wobei der Nutzerbereich (75) einen Proximalbereich zur Längsseitenkante (40) und einen Distalbereich zur Längsseitenkante (40) aufweist; wobei der Proximalbereich ein haftendes Befestigungsteil (63) umfaßt und der Distalbereich ein mechanisch haftendes Teil (62) und eine Griffzunge (61) umfaßt, wobei das haftende Befestigungsteil (63) und das mechanische Befestiungsteil (62) mit ihrer Befestigungsfläche jeweils in der gleichen Richtung orientiert sind wie die Innenfläche (44); wobei die Griffzunge (61) an einer seitlich äußeren Kante (72) des Nutzerbereichs (75) angeordnet ist;
- ein mechanisch befestigendes Anlageteil (64) zur Zusammenwirkung mit dem mechanischen Befestigungsteil (62) des haft-mechanischen Befestigungsbandstreifens (60), wobei das Anlageteil (64) in dem seitlich zweiten Endbereich (50) angeordnet ist und mit der Außenfläche (46) des absorbierenden Artikels (20) verbunden ist;
- der Griffzunge (61), die sich über einen Teil des Distalbereichs des Nutzerbereichs (75) erstreckt und durch Falten der seitlich äußeren Kante (72) des Nutzerbereichs (75) zurück auf sich selbst vorgesehen ist, dadurch gekennzeichnet, daß
sich die Griffzunge (61) über einen Teil des mechanischen Befestigungsteils (62) erstreckt.

2. Absorbierender Einwegartikel (20) nach Anspruch 1, in welchem die Griffzunge (61) mit dem Proximalbereich des Nutzerbereichs (75) kontinuierlich ausgebildet ist.

3. Absorbierender Einwegartikel (20) nach einem der vorstehenden Ansprüche, in welchem der absorbierende Artikel (20) ferner ein Abziehband (65) umfaßt, wobei das Abziehband (65) auf der Innenfläche (44) des absorbierenden Artikels (20) angeordnet ist, um mit dem haft-mechanischen Befesfigungsbandstreifen (60) während der Speicherung und des Transports des absorbierenden Artikels (20) zusammenzuwirken.

4. Absorbierender Einwegartikel (20) nach Anspruch 3, in welchem das Abziehband (65) mit der Innenfläche (44) des absorbierenden Artikels (20) durch ein Haftmittel verbunden ist.

5. Absorbierender Einwegartikel (20) nach einem der vorstehenden Ansprüche, in welchem der Proximalbereich und der Distalbereich des Nutzerbereichs (75) kontinuierlich ausgebildet sind und eine kontinuierliche Haftschicht haben, um das haftende Befestigungsteil (63) in dem Proximalbereich zu bilden, und das mechanische Befestigungsteil (62) mit dem haftmechanischen Befestigungsbandstreifen (60) durch die kontinuierliche Haftschicht verbunden ist.

6. Absorbierender Einwegartikel (20) nach Anspruch 5, in welchem der Nutzerbereich (75) und der Herstellerbereich (74) kontinuierlich ausgebildet sind, wobei der Nutzerbereich (75) und der Herstellerbereich (74) kontinuiedich ausgebildet sind und eine kontinuierliche Haftschicht haben, wobei der Herstellerbereich (74) mit der Außenfläche (46) des absorbierenden Artikels (20) durch die kontinuierliche Haftschicht befestigt ist.

7. Absorbierender Einwegartikel (20) nach einem der vorstehenden Ansprüche, in welchem das mechanische Befestigungsteil (62) hakenförmige Eingriffmittel umfaßt und das mechanisch befestigende Anlageteil schleifenförmige Eingriffsmittel umfaßt, um sich während der Benutzung des absorbierenden Artikels (20) mit den hakenförmigen Eingriffsmitteln zu verbinden.

8. Absorbierender Einwegartikel (20) nach einem der vorstehenden Ansprüche, in welchem das mechanisch befestigende Anlageteil (64) mit der Außenfläche (46) des absorbierenden Artikels (20) durch ein Haftmittel verbunden ist und das mechanisch befestigende Anlageteil (64) als ein einheitliches Stück vorgesehen ist.

9. Absorbierender Einwegartikel (20) nach einem der vorstehenden Ansprüche, in welchem der absorbierende Einwegartikel (20) eine absorbierende Einwegwindel (20) ist.

## Revendications

1. Article absorbant jetable présentant une surface intérieure (44) et une surface extérieure (46) des bords latéraux longitudinaux (40) dans la direction longitudinale, un premier et un deuxiême bord d'extrémité latéral (42) dans la direction latérale une première région d¹extrémité (48) et une deuxième région d'extrémité (50), ledit article absorbant comprenant
- une partie de corps (22) définie par lesdits bords latéraux longitudinaux (40) et lesdits premier et deuxième bords d'extrémité latéraux (42);
- une patte à ruban de fixation adhésive-mécanique (60) présentant une région du fabricant (74) et une région de l'utilisateur (75) ; ladite patte à ruban (60) étant placée dans ladite première région d'extrémité (48) latérale afin que ladite région du fabricant (74) soit réunie audit article absorbant (20) et que ladite région de l'utilisateur (75) s'étende au-delà dudit bord latéral longitudinal (40) dudit article absorbant (20); ladite région de l'utilisateur (75) présentant une région proximale par rapport audit bord latéral longitudinal (40) et une région distale par rapport audit bord latéral longitudinal (40) ; ladite région proximale comprenant un élément de fixation adhésif (63), et ladite région distale comprenant un élément de fixation mécanique (62) et une patte de préhension (61), ledit élément de fixation adhésif (63) et ledit élément de fixation mécanique (62) ayant chacun leur surface de fixation orientée dans la même direction que ladite surface intérieure (44); ladite patte de préhension (61) étant placée au niveau du bord latéralement extérieur (72) de ladite région de l'utilisateur (75);
- un élément de réception de fixation mécanique (64) destiné à coopérer avec ledit élément de fixation mécanique (62) de ladite patte à ruban de fixation adhésive-mécanique (60), ledit élément de réception (64) étant placé dans ladite deuxième région d'extrémité (50) latérale et réuni à ladite surface extérieure (46) dudit article absorbant (20) ; et
- ladite patte de préhension (61) s'étendant sur une partie de la région distale de ladite région de l'utilisateur (75) et étant réalisée en repliant ledit bord latéralement extérieur (72) de la région de l'utilisateur (75) sur lui-même,
caractérisé en ce que
ladite patte de préhension (61) s'étend sur une partie dudit élément de fixation mécanique (62).

2. Article absorbant jetable (20) selon la revendication 1, dans lequel ladite patte de préhension (61) est continue avec ladite région proximale de ladite région de l'utilisateur (75).

3. Article absorbant jetable (20) selon l'une quelconque des revendications précédentes, dans lequel ledit article absorbant (20) comprend, en outre, un ruban de dégagement (65), ledit ruban de dégagement (65) étant placé sur ladite surface intérieure (44) dudit article absorbant (20) pour coopérer avec ladite patte à ruban de fixation adhésive-mécanique (60) durant le stockage et le transport dudit article absorbant (20).

4. Article absorbant jetable (20) selon la revendication 3, dans lequel ledit ruban de dégagement (65) est réuni à ladite surface intérieure (44) dudit article absorbant (20) par un adhésif.

5. Article absorbant jetable (20) selon l'une quelconque des revendications précédentes, dans lequel ladite région proximale et ladite région distale de ladite région de l'utilisateur (75) sont continues et ont une couche adhésive continue pour former ledit élément de fixation adhésif (63) de ladite région proximale, et ledit élément de fixation mécanique (62) étant réuni à ladite patte à ruban de fixation adhésive-mécanique (60) par ladite couche adhésive continue.

6. Article absorbant jetable (20) selon la revendication 5, dans lequel ladite région de l'utilisateur (75) et ladite région du fabricant (74) sont continues, ladite région de l'utilisateur (75) et ladite région du fabricant (74) étant continues et présentant une couche adhésive continue, ladite région du fabricant (74) étant assujettie à ladite surface extérieure (46) dudit article absorbant (20) par ladite couche adhésive continue.

7. Article absorbant jetable (20) selon l'une quelconque des revendications précédentes, dans lequel ledit élément de fixation mécanique (62) comprend un moyen de prise de type à crochets, et ledit élément de réception de fixation mécanique comprend un élément de prise de type à boucles pour venir en prise avec ledit moyen de prise de type à crochets durant l'utilisation dudit article absorbant (20).

8. Article absorbant jetable (20) selon l'une quelconque des revendications précédentes, dans lequel ledit élément de réception de fixation mécanique (64) est réuni à ladite surface extérieure (46) dudit article absorbant (20) par un adhésif, et ledit élément de réception de fixation mécanique (64) est prévu en tant que pièce unitaire.

9. Article absorbant jetable (20) selon l'une quelconque des revendications précédentes, dans lequel ledit article absorbant jetable (20) est une couche absorbante jetable (20).
